(19) **Europäisches Patentamt / European Patent Office / Office européen des brevets**

(11) **EP 2 829 218 B1**

(12) **EUROPEAN PATENT SPECIFICATION**

(45) Date of publication and mention
of the grant of the patent:
**03.05.2017 Bulletin 2017/18**

(21) Application number: **13764231.0**

(22) Date of filing: **15.03.2013**

(51) Int Cl.:
*A61B 1/04* *(2006.01)*          *A61B 1/00* *(2006.01)*
*G02B 23/24* *(2006.01)*       *G06T 7/00* *(2017.01)*
*A61B 90/00* *(2016.01)*       *A61B 34/20* *(2016.01)*
*A61B 1/313* *(2006.01)*

(86) International application number:
**PCT/JP2013/057392**

(87) International publication number:
**WO 2013/141155 (26.09.2013 Gazette 2013/39)**

(54) **IMAGE COMPLETION SYSTEM FOR IN-IMAGE CUTOFF REGION, IMAGE PROCESSING DEVICE, AND PROGRAM THEREFOR**

BILDVERVOLLSTÄNDIGUNGSSYSTEM FÜR EINEN BILDAUSSCHNITTBEREICH, BILDVERARBEITUNGSVORRICHTUNG UND PROGRAMM DAFÜR

SYSTÈME D'EXÉCUTION D'IMAGE POUR UNE RÉGION TRONQUÉE DANS L'IMAGE, DISPOSITIF DE TRAITEMENT D'IMAGE ET PROGRAMME ASSOCIÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2012 JP 2012061285**

(43) Date of publication of application:
**28.01.2015 Bulletin 2015/05**

(73) Proprietors:
• **WASEDA UNIVERSITY**
**Shinjuku-ku**
**Tokyo 169-8050 (JP)**
• **Kyushu University,**
**National University Corporation**
**Fukuoka-shi, Fukuoka 812-8581 (JP)**

(72) Inventors:
• **FUJIE, Masakatsu**
**Tokyo 169-8050 (JP)**
• **KOBAYASHI, Yo**
**Tokyo 169-8050 (JP)**
• **KAWAMURA, Kazuya**
**Tokyo 169-8050 (JP)**

• **SENO, Hiroto**
**Tokyo 169-8050 (JP)**
• **NISHIO, Yuya**
**Tokyo 169-8050 (JP)**
• **HASHIZUME, Makoto**
**Fukuoka-shi**
**Fukuoka 812-8581 (JP)**
• **IEIRI, Satoshi**
**Fukuoka-shi**
**Fukuoka 812-8581 (JP)**
• **TOYODA, Kazutaka**
**Fukuoka-shi**
**Fukuoka 812-8581 (JP)**

(74) Representative: **Grünecker Patent- und Rechtsanwälte**
**PartG mbB**
**Leopoldstraße 4**
**80802 München (DE)**

(56) References cited:
**EP-A1- 2 425 761          WO-A1-2011/114731**
**WO-A1-2011/118287       JP-A- 2004 065 804**
**JP-A- 2005 021 353        JP-A- 2006 198 032**
**JP-A- 2007 152 027        US-A1- 2012 271 102**

**Description**

Technical Field

The present invention relates to an image completion system for an in-image cutoff region, an image processing device, and program therefor, and more particularly, relates to an image completion system for an in-image cutoff region, an image processing device, and a program therefor, which completes image information on an object space imaged in the state where a portion thereof is hidden by a predetermined member, with other image information imaged in another line-of-sight direction.

Background Art

In recent years, minimally invasive surgery which does not need a large incision and reduces loads imposed on a patient is widespread, and as the minimally invasive surgery, endoscopic surgery is known. The endoscopic surgery is surgery in which a rod-shaped surgical instrument provided with a scalpel, forceps, a puncture needle, or the like on the tip side thereof, and an endoscope are inserted in the body through holes opened at portions on the body surface of a patient, and an operator treats an affected area by manipulating the surgical instrument from the outside of the body of the patient. Such endoscopic surgery includes a mode in which a surgical instrument is directly manipulated by the hands of an operator, as well as a mode assisted by a surgery assistant robot in which a surgical instrument is moved by the operation of a robot arm.

In the above-described endoscopic surgery, however, the operator cannot directly see the affected area and the surrounding area thereof but can visually confirm the affected area with only an endoscopic image on a monitor, and thus the operator is problematically restricted in the visual field. In particular, with regard to an endoscopic image, when a surgical instrument is displayed, an internal space existing on the depth side of the surgical instrument is hidden by the surgical instrument and cannot be visually confirmed. Such a case may be able to be dealt with by the manipulation of, for example, changing the attitude of the endoscope, but the operation during the surgery is troublesome. In addition, the surgical instrument is often close to an affected area during the surgery and is still often displayed somewhere in the endoscopic image even when the attitude of the endoscope is changed. The existence of a cutoff region due to the surgical instrument thus often makes the visual field even narrower, which makes accurate grasping of the space near the affected area further difficult. For this reason, when there is a dangerous site such as a vessel and a nerve which the surgical instruments should not touch, in the cutoff region, the surgical instrument may be unintendedly touch the dangerous site to cause an accident such as bleeding to occur.

Now, Patent Literature 1 discloses a surgery supporting device for processing three-dimensional image data imaged by an MRI (Magnetic Resonance Imaging system) or the like and superimposing the processed three-dimensional image data onto an endoscopic image. This surgery supporting device is configured to extract a specified region in the three-dimensional image to create segmentation image data, subject the segmentation image data to a projecting process to create a surgery assistant image, and superimpose the surgery assistant image onto the endoscopic image.

In addition, Patent Literature 2 discloses an image processing device for establishing correspondences between a stereoscopic endoscope picture imaged during a surgery and a three-dimensional image obtained from image data imaged by an MRI or the like prior to the surgery, and performing registration between the images to compose the images and display the composite image. This image processing device is configured to, when a portion of one of left and right stereoscopic endoscope pictures is cut off by a surgical instrument, geometrically restore feature points of a tissue existing on the back side of the surgical instrument so as to grasp the three-dimensional position of the tissue existing on the back side of the surgical instrument.

EP 2 425 761 discloses a medical apparatus including two image pickup sections and an image processing device.

WO 2011118287 discloses an image processing device which replaces present image information on a treatment tool with previously stored corresponding image information in which a treatment tool is not yet visible.

JP 2004-065804 discloses an image processing device which superimposes previously stored image information as halftone image information on present image information.

Citation List

Patent Literature

   Patent Literature 1: Japanese Patent Laid-Open No. 2007-7041
   Patent Literature 2: Japanese Patent Laid-Open No. 11-309

Summary of Invention

Technical Problem

In the above-described surgery supporting device of Patent Literature 1, however, it does not mean that, when the surgical instrument is displayed in the endoscopic image, a cutoff region thereof in a depth direction of the endoscopic image is automatically identified to obtain image information on an internal space in the cutoff region, and thus the surgery supporting device cannot solve the above-described problem of the restriction of the visual field of an operator due to the existence of a surgical instrument in an endoscopic image.

In addition, the above-described image processing device of Patent Literature 2 is subject to a condition that a stereoscopic endoscope picture in which a surgical instrument is not displayed is first obtained and the position and the attitude of the stereoscopic endoscope is not changed from those at that time during the surgery, in order to identify, in the stereoscopic endoscope, the three-dimensional position of the tissue on the back side that is hidden by the surgical instrument or the like. It is therefore needed an operation to retract the surgical instruments into a place which is not displayed in the endoscopic picture every time the attitude of the stereoscopic endoscope is changed, which obstructs an smooth operation of the surgery. Furthermore, since the three-dimensional image that has been imaged by an MRI or the like prior to the surgery is superimposed onto the endoscopic picture, if the state of the an internal space imaged in the endoscopic picture changes due to the movement of an organ or the like displayed in the endoscopic picture during the surgery, the correspondences of the same portion cannot be established between the endoscopic picture obtained in real time and the three-dimensional image representing a past state of the internal space having been obtained by the MRI or the like prior to the surgery, and thus the three-dimensional image cannot be superimposed onto the endoscopic picture.

The present invention is devised in light of such problems, and has an object to provide an image completion system for an in-image cutoff region, an image processing device, and a program therefor which, with respect to an image in which a predetermined object space is imaged, if there is a cutoff region where a portion of the image is cut off by a predetermined member, can complete image information on the object space in the cutoff region without troublesome operation even when the condition of the object space changes.

Solution to Problem

In order to achieve the above-described object, the present invention provides an image completion system according to claim 1, an image processing device according to claim 6 and a program according to claim 7.

Advantageous Effect of Invention

According to a first aspect, a member such as a surgical instrument is imaged in a main image together with an object space, and when image information on a portion of the object space is hidden by the surgical instrument, image information on the depth side of the member in the hidden portion is completed with image information on a real-time completing image, and a composite image that looks as if the member were seen through can be obtained with respect to the main image in real time. As a result, a cutoff region by the member is cancelled by image processing, and the reduction of a visual field in the main image due to the existence of the cutoff region is ameliorated, which allows the visual field to be substantially expanded.

Brief Description of Drawings

[Figure 1] Figure 1 is a schematic system configuration diagram of an image completion system according to the present embodiment.
[Figure 2] Figures 2 (A) to (F) are diagrams for illustrating a procedure for obtaining a composite image of an operating field image V1 and a completing image V2.
[Figure 3] Figure 3 is a schematic view for illustrating conversion between coordinate systems.
[Figure 4] Figure 4 (A) is a diagram showing images for illustrating in-image movement of a set point $P_i$, and Figure 4 (B) is a diagram showing an image enlarging a portion of Figure 4 (A) for illustrating in-image movement of an unset point $P_p$.

Description of Embodiment

The embodiment according to the present invention will be described below with reference to the drawings.

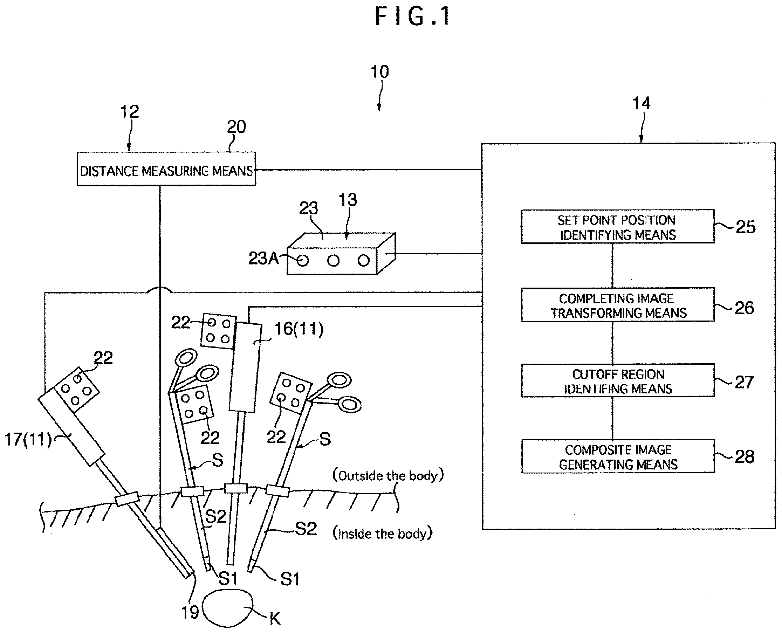

Figure 1 shows a schematic system configuration diagram of an image completion system for an in-image cutoff region according to the present embodiment. In this drawing, an image completion system 10 according to the present embodiment is a system for completing a endoscopic image used in endoscopic surgery in which a surgery is performed by manipulating treating parts S1 such as a scalpel or forceps attached to the tips of surgical instruments S, from the outside of a body.

This image completion system 10 includes imaging device 11 for imaging an image of an object space being an internal space to be monitored formed by an organ K including an affected area to be treated and the surrounding areas thereof, a distance measuring device 12 for measuring separating distances between a predetermined reference point and a large number of set points that are virtually set to objects in the object space, a three-dimensional position measuring device 13 for measuring three-dimensional position of the imaging device 11, and an image processing device 14 for processing the image obtained by the imaging device 11.

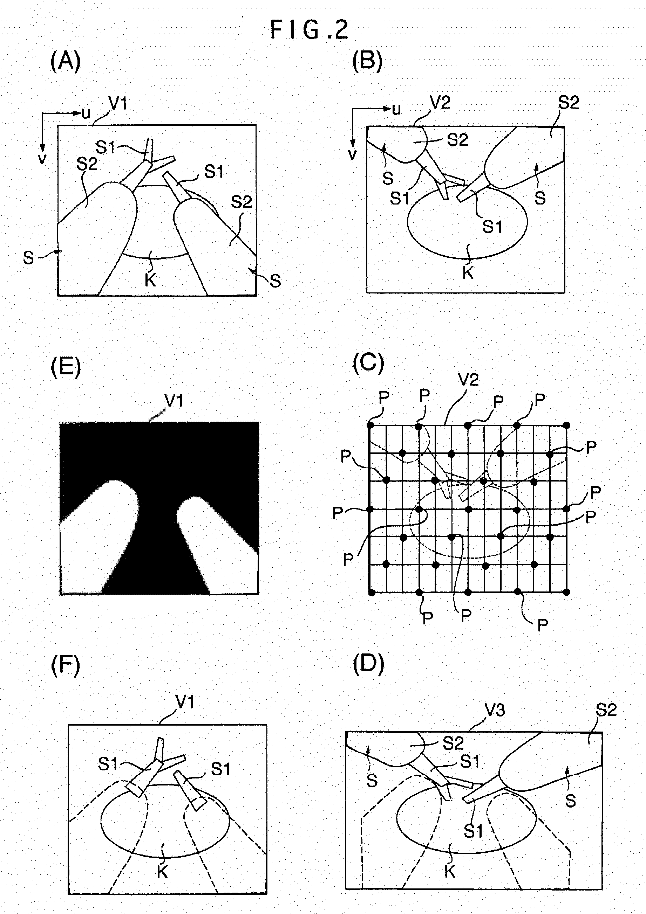

The imaging device 11 is configured by a single lens operating field endoscope 16 (main imaging device) for obtaining an operating field image V1 (refer to Figure 2(A)) to be a main image composing an endoscopic image of a treating region that an operator looks at in the surgery, and a single lens completing-purpose endoscope 17 (completing-purpose imaging device) for obtaining a completing image V2 (refer to Figure 2 (B)) for completing the operating field image. The operating field endoscope 16 is configured to image an image of a desired object space under the instructions or manipulations of the operator. The completing-purpose endoscope 17 is configured so as to be enabled to image an image of the object space from a line-of-sight direction different from that of the operating field endoscope 16, and may be configured so as to be enabled to move following the movement of the operating field endoscope 16 in an integrated manner, or may be configured so as to be enabled to move with the operating field endoscope 16 in a relative manner. Note that the completing-purpose endoscope 17 is disposed so as to be enabled to image a depth-side region of the object space that is hidden by surgical instruments S existing in the operating field image V1 imaged by the operating field endoscope 16 in the operating field image V1.

As the distance measuring device 12, for example, there are used devices having a well-known structure disclosed in Japanese Patent Laid-Open No. 2010-220787 or the like, and the distance measuring device 12 includes stereo camera 19 that can obtain a stereo image, and distance measuring means 20 that searches for a corresponding point between a pair of stereo images imaged by the stereo camera 19 and calculates distances from the end of the stereo camera 19 to the corresponding point, by a stereo matching method. Note that the descriptions of the structure and the algorithm of the distance measuring device 12 in detail will be omitted since well-known techniques are used therefor, which is not an essential part of the present invention.

Here, the stereo camera 19 is provided integrally with completing-purpose endoscope 17, and is configured so as to be enabled to obtain an almost entire stereo image of a space imaged by the completing-purpose endoscope 17.

In the distance measuring means 20, as schematically shown in Figure 2 (C), a large number of set points P are automatically set on the surface of objects imaged by the completing-purpose endoscope 17, and with respect to the set points P, distances from the end of the stereo camera 19 are calculated and set of three-dimensional coordinates (three-dimensional positions) are identified (detected) in a stereo camera coordinate system having an origin being a predetermined point of stereo camera 19. Here, the set points P are not limited in particular, and the number thereof may be at least three, and in the present embodiment, a large number of set points P are set on the objects imaged by the completing-purpose endoscope 17 with predetermined horizontal and vertical intervals in the screen. Note that one of the cameras of the stereo camera 19 may be also used as the completing-purpose endoscope 17.

The three-dimensional position measuring device 13 includes markers 22, at least three of which are attached to members to be subjected to position measurement, and a body 23 including light receiving parts 23A for receiving infrared rays emitted by the markers 22. As the three-dimensional position measuring device 13, there are used devices having a well-known configuration which can detect the three-dimensional positions of the markers 22 by tracking the infrared rays following the movements of the markers 22. The description of the structure in detail will be omitted since it is not an essential part of the present invention. Note that as the three-dimensional position measuring device 13, devices making use of various principles or structured can be alternatively used as long as they can detect the three-dimensional positions of the members to be subjected to the position measurement.

Here, the markers 22 are attached to the rear end portion of each surgical instruments S, the operating field endoscope 16, and the completing-purpose endoscope 17, the rear end portions positioned outside the body in the surgery, and the body 23 identifies the sets of three-dimensional coordinates (positions) with respect to the rear end portions, in the reference coordinate system having an origin being a predetermined point. In addition, the sets of three-dimensional coordinates of components that do not move relatively with respect to the rear end portions are calculated from the sets of three-dimensional coordinates of the rear end portions through mathematical operations performed in the body 23 because the surgical instruments S, the operating field endoscope 16, and the completing-purpose endoscope 17 each have a known shape that has been identified in advance. Note that if the operating field endoscope 16 and the completing-purpose endoscope 17 are integrated in such a manner as not to relatively move, the markers 22 may be provided to only one of them. In addition, in the present embodiment, since the completing-purpose endoscope 17 and the stereo camera 19 of the distance measuring device 12 are provided in such a manner as not to relatively move, when the positions of the components of the completing-purpose endoscope 17 are calculated by the three-dimensional position measuring device 13, the positions of the components of the stereo camera 19 are also identified automatically. It is thereby possible to convert the sets of three-dimensional coordinates of the set points P in the stereo camera coordinate system calculated by the distance measuring device 12 into the sets of three-dimensional coordinates in the reference coordinate system on the basis of the measurement result from the three-dimensional position measuring device 13.

Note that if the stereo camera 19 can be relatively move with respect to all the surgical instruments S, the operating field endoscope 16, and the completing-purpose endoscope 17, the markers 22 are attached also to the rear end portion of the stereo camera 19.

The image processing device 14 is configured by a computer formed by a processing unit such as a CPU and a storage such as a memory and a hard drive, and includes a program installed for causing the computer to function as the following means.

This image processing device 14 is configured to obtain, from the completing image V2, image information on cutoff regions in the object space hidden on the depth side thereof by the surgical instruments S displayed in the operating field image V1, and to replace image information on the cutoff regions in the operating field image V1 with the obtained image information or superimposing the obtained image information onto the image information on the cutoff regions in the operating field image V1 so as to perform a process of generating a composite image in which the cutoff regions are completed by the completing image.

Specifically, the image processing device 14 includes set point position identifying means 25 for identifying, with respect to the set points P, sets of three-dimensional coordinates (three-dimensional positions) in the reference coordinate system on the basis of the measurement results from the distance measuring device 12 and the three-dimensional position measuring device 13 and for calculating sets of in-screen coordinates (sets of two-dimensional coordinates) in the screen coordinate system in the operating field image V1 and sets of in-screen coordinates (two-dimensional coordinates) in the screen coordinate system in the completing image V2, completing image transforming means 26 for generating a transformed image V3 (refer to Figure 2 (D)) obtained by moving image information on points (pixels) in the completing image so as to convert the image information in the completing image V2 into that in a line-of-sight direction of the operating field endoscope 16 on the basis of the sets of in-screen coordinates calculated by the set point position identifying means 25, cutoff region identifying means 27 for identifying cutoff regions occupied by rod-shaped main body parts S2 that are behind the treating parts S1 of the surgical instruments S in the operating field image V1, and composite image generating means 28 for identifying corresponding regions (dotted-lined regions in Figure 2 (D)) corresponding to the cutoff regions in the transformed image V3 and for replacing the image information on the cutoff regions in the operating field image V1 with image information on the corresponding regions in the transformed image V3 or superimposing the image information on the corresponding regions in the transformed image V3 onto the image information on the cutoff regions in the operating field image V1 to generate a composite image of the operating field image V1 and the completing image V2.

The procedure of the image completion in the image processing device 14 will be described below.

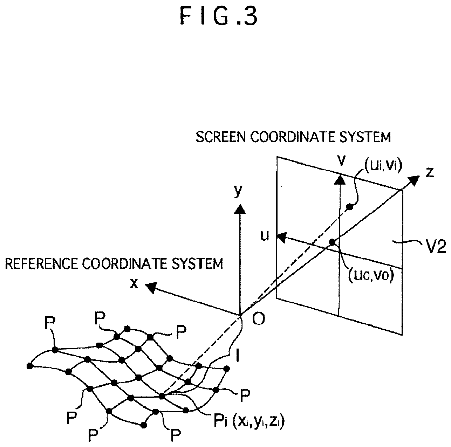

First, the set point position identifying means 25 converts the sets of three-dimensional coordinates of the set points P in the stereo camera coordinate system calculated by the distance measuring device 12 into the sets of three-dimensional coordinates in the reference coordinate system (refer to Figure 3), on the basis of the measurement result from the three-dimensional position measuring device 13. The set point position identifying means 25 then calculates the sets of in-screen coordinates (two-dimensional coordinates) of the set points P in the completing image V2 by the following well-known formulae that have been stored in advance. Note that, the reference coordinate system being a three-dimensional coordinate system is set such that a z-axis direction thereof matches the optical axis direction of the completing-purpose endoscope 17.

[Formula 1]

$$u_i = f k_u \frac{x_i}{z_i} + u_0 \qquad (1)$$

$$v_i = f k_v \frac{y_i}{z_i} + v_0 \qquad (2)$$

In the above formulae, a set of coordinates ($x_i$, $y_i$, $z_i$) is a set of three-dimensional coordinates of each set point $P_i$ (i=1 to n) in the reference coordinate system. In addition, in the formulae (1) and (2), a set of coordinates ($u_i$, $v_i$) is a set of in-screen coordinates of a set point $P_n$ in the screen coordinate system in the completing image V2, which is a set of two-dimensional coordinates in the horizontal direction in the screen and the vertical direction in the screen. In addition, f is a focal distance of the operating field endoscope, $k_u$ is a screen resolution of the completing-purpose endoscope 17 in the horizontal direction in the screen, $k_v$ is a screen resolution of the completing-purpose endoscope 17 in the vertical direction in the screen, and a set of coordinates ($u_0$, $v_0$) is a set of coordinates of a point in the horizontal direction in the screen and the vertical direction in the screen, at which the optical axis crosses the image surface of the completing image V2. Here, f, $k_u$, $k_v$, $u_0$, and $v_0$ are constants that have been specified in accordance with the specification or the state of disposition of the completing-purpose endoscope 17, and stored in advance.

Next, the sets of coordinates ($x_i$, $y_i$, $z_i$) of the set points $P_i$ in the reference coordinate system are converted into sets of three-dimensional coordinates ($x'_i$, $y'_i$, $z'_i$) having a reference being a predetermined position of the operating field endoscope 16, on the basis of a relative position relationship between the operating field endoscope 16 and the completing-purpose endoscope 17 based on the measurement result from the three-dimensional position measuring device 13, and further converted into set of in-screen coordinates ($u'_i$, $v'_i$) of the set point $P_i$ in the operating field endoscope 16 by formulae similar to the above formulae (1) and (2).

Next, in the completing image transforming means 26, on the basis of the sets of in-screen coordi-

nates $(u'_i, v'_i)$ of the set points $P_i$ in the operating field image V1 and the sets of in-screen coordinates $(u_i, v_i)$ of the set points $P_i$ in the completing image V2, pieces of image information on the points in the completing image V2 are moved, in the completing image V2, to positions corresponding to the sets of in-screen coordinates in the operating field image V1 at which the same portions of the points in the completing image V2 are displayed, whereby the transformed image V3 for the completing image V2 is generated.

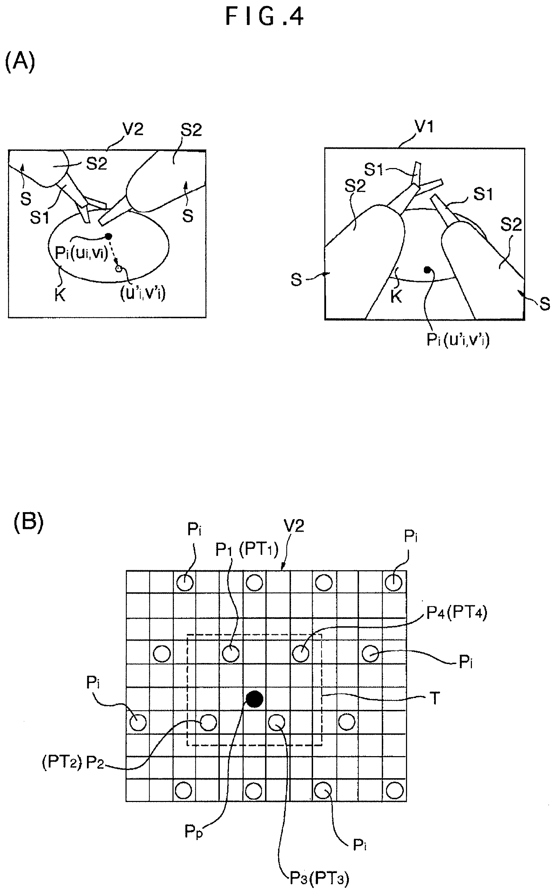

In other words here, first, as shown in Figure 4 (A), the piece of image information on the set point $P_i$ at the set of in-screen coordinates $(u_i, v_i)$ in the completing image V2 is moved in the completing image V2 such that the set of in-screen coordinates $(u_i, v_i)$ become a set of in-screen coordinates same as the set of in-screen coordinates $(u'_i, v'_i)$ of the corresponding set point $P_i$ in the operating field image V1. Next, as shown in Figure 4 (B), pieces of image information on unset points $P_p$ (p=1, 2, ...) (solid black circles in the drawing: only one of them is shown) that are remaining parts except for the set points $P_i$ (solid while circles in the drawing) in the completing image V2 are moved by a weighted average as follows. Note that, in Figure 4 (B), the set points $P_i$ are shown by the solid while circles in the drawing. In contrast, only one solid black circle is shown with respect to the unset point $P_p$ for a reason of preventing the drawing from being complicated, but actually the unset points $P_p$ exist at every pixel portion in the screen except for the set points $P_i$.

First, a virtual region T that has a certain range smaller than the entire completing image V2 is set, and the set points $P_i$ existing in the virtual region T are identified around the unset point $P_p$. In the example in Figure 4 (B), there are four set points $P_i$ from $P_1$ to $P_4$ existing in the virtual region T.

Next, the following weight coefficients $W_i$ are calculated in such a manner as to correspond to the set points $P_i$ existing in the virtual region T. Specifically, a separating distance with respect to the unset point $P_p$ is calculated for each set point $P_i$ existing in the virtual region T, and the weight coefficient $W_i$ is calculated from the separating distance using a preset arithmetic formula. These weight coefficients $W_i$ are set so as to be in inverse proportion to the separating distances.

Next, movement vectors $T(u_p, v_p)$ for the unset points $P_p$ are calculated by the following formula, respectively. Note that, here, a number N of set points $P_i$ existing in the virtual region T are defined as set points $PT_j$ (j=1, 2, ..., N), the movement vectors in the completing image V2 that are identified with respect to the set points $PT_j$ by the above-described procedure are defined as $T(u_j, v_j)$, and the above-described weight coefficients corresponding to the separating distances from the unset points $P_p$ are defined as $W_j$.

[Formula 2]

$$T(u_p, v_p) = \frac{\sum_{j=1}^{N} W_j T(u_j, v_j)}{\sum_{j=1}^{N} W_j} \qquad (3)$$

The pieces of image information on the unset points $P_p$ in the completing image V2 are thereafter moved in the screen of the completing image V2 according to the amount and the direction of the movement based on the calculated movement vectors $T(u_p, v_p)$. As a result, the transformed image V3 is generated in such a manner that the pieces of image information on the set points $P_i$ and the unset points $P_p$ in the completing image V2 are moved in the same screen so as to convert the completing image V2 into that in the line-of-sight direction of the operating field endoscope 16.

Note that, in the completing image transforming means 26, the movement vectors $T(u_p, v_p)$ of the pieces of image information on the unset points $P_p$ are calculated by the weighted average, but the movement vectors $T(u_p, v_p)$ may be calculated by other methods such as B-spline interpolation on the basis of pieces of position information on the set points $P_i$.

Next, in the cutoff region identifying means 27, the positions of the cutoff regions occupied by the main body parts S2 in the operating field image V1 are identified as follows. That is, the three-dimensional position measuring device 13 calculates the sets of three-dimensional coordinates of the parts of the surgical instruments S1 in the reference coordinate system. These sets of three-dimensional coordinates are then converted into the sets of in-screen coordinates (two-dimensional coordinates) in the screen coordinate system of the operating field image V1 using arithmetic formulae similar to those in the description of the set point position identifying means 25, and the positions of the cutoff regions in the operating field image V1 are identified. Note that, the identification of the cutoff regions is not limited to the above-described method, and well-known methods may be used in which predetermined colors are applied to the main body parts S2 and the pieces of image information on the operating field image V1 are distinguished on the basis of the colors to identify the cutoff regions.

Thereafter, in the composite image generating means 28, a composite image is generated by performing the following mask process. That is, first, as shown in Figure 2 (E), a mask is generated by extracting the cutoff regions identified in the operating field image V1. Then, ranges of the sets of in-screen coordinates in the transformed image V3 (the drawing (D)) that match ranges of the in-screen coordinates of the cutoff regions in the operating field image V1 are identified as corresponding regions (dotted-lined regions in the drawing (D)) by the generated mask, and the pieces of image information on these corresponding regions are extracted. The pieces of image information on the cutoff regions in the operating field image V1 are thereafter superimposed or replaced with the pieces of image information on the corresponding regions, and the composite image shown in the drawing (F) is thereby generated.

The composite image is an image having the operating field image V1 as a base, in which the pieces of image information on the depth sides of the main body parts S2 are completed by the completing image V2 from the completing-purpose endoscope 17 as if the main body parts S2 of the surgical instruments S displayed in the operating field image V1 are made transparent or translucent. Therefore, in the composite image, only the treating parts S1 being the tips of the surgical instruments S imaged in the operating field image V1 are left, and the internal space except for the treating parts S1 that an operator needs during the surgery can be imaged in the operating field image V1, which allows the operating field of the endoscopic image to be substantially expanded.

Note that, in the above-described embodiment, there has been illustrated and described the image completion system 10 for performing image processing to the endoscopic image in endoscopic surgery, but the present invention is not limited to this, and can be applied to image processing to an endoscopic image from a surgery assistant robot for assisting endoscopic surgery, as well as can be applied to, for example, image processing for performing a remote control of a robot arm while obtaining an image from an imaging device such as a camera in an operation in a working space such as a reactor of a nuclear power plant that a human cannot enter and directly see. In this case, the replacement of the above-described surgical instrument S with a member such as a robot arm that has been specified in advance and the application of an algorithm similar to the above make it possible to implement an image completion system that meets the use.

In addition, the configuration of each part of the device in the present invention is not limited to the illustrated exemplary configurations, and can be subjected to various modifications as long as it exhibits substantially similar effects.

Industrial Applicability

The present invention is industrially applicable as a system for completing a restricted visual field by using an imaging device for obtaining an image of the inside of a space that a human cannot directly see.

Reference Signs List

    10 image completion system
    11 imaging device
    12 distance measuring device
    13 three-dimensional position measuring device
    14 image processing device
    16 operating field endoscope (main imaging device)
    17 completing-purpose endoscope (completing-purpose imaging device)
    25 set point position identifying means
    26 completing image transforming means
    27 cutoff region identifying means
    28 composite image generating means
    P set point
    S surgical instrument (member)
    V1 operating field image (main image)
    V2 completing image

Claims

1. An image completion system for an in-image cutoff region, comprising:

    a main imaging device configured for obtaining a main image in which an object space to be monitored is imaged;
    a completing-purpose imaging device configured for obtaining a completing image used for completing the main image by imaging the object space in a line-of-sight direction different from that of the main imaging device; and
    an image processing device configured for completing a portion of the main image with the completing image on the basis of three-dimensional positions of set points which are set in the object space and three-dimensional positions of the main imaging device and the completing-purpose imaging device,
    a distance measuring device configured for measuring separating distances between the set points and a predetermined reference point; and
    a three-dimensional position measuring device configured for measuring three-dimensional positions of the main imaging device and the completing-purpose imaging device,
    wherein the main imaging device and the completing-purpose imaging device are provided so as to image the object space almost simultaneously,
    wherein the image processing device is configured for obtaining from the completing image, image information on a cutoff region in the object space that is hidden behind on a depth side of a member having a known shape by imaging the member in the main image together with the object space, on the basis of information on the three-dimensional positions that are continuously detected, and for replacing image information on the member in the main image with the obtained image information or superimposes the obtained image information onto the image information on the member in the main image so as to generate a composite image in which the cutoff region is completed with the completing image, and

wherein the image processing device is configured to complete a portion of the main image with the completing image on the basis of measurement results from the distance measuring device and the three-dimensional position measuring device.

2. The image completion system for an in-image cutoff region according to claim 1, wherein the image processing device includes:

set point position identifying means for identifying, with respect to the set points, sets of in-screen coordinates in a screen coordinate system in the main image and sets of in-screen coordinates in a screen coordinate system in the completing image on the basis of a detection result of the three-dimensional positions;

completing image transforming means for generating, on the basis of the sets of in-screen coordinates, a transformed image in which pieces of image information on points in the completing image are moved in a screen of the completing image such that the completing image is converted into that in a line-of-sight direction of the main imaging device;

cutoff region identifying means for identifying a position of the cutoff region in the main image; and

composite image generating means for generating the composite image by replacing image information on the cutoff region with image information on a corresponding region that corresponds to the cutoff region in the transformed image or superimposing the image information on the corresponding region onto the image information on the cutoff region.

3. The image completion system for an in-image cutoff region according to claim 1, wherein at least three set points are set in the object space.

4. The image completion system for an in-image cutoff region according to claim 1, wherein the main imaging device and the completing-purpose imaging device are provided so as to independently move to image the object space.

5. The image completion system for an in-image cutoff region according to claim 2, wherein the completing image transforming means generates the transformed image by moving the pieces of image information on the completing image such that the set points in the completing image match sets of in-screen coordinates of the same set points existing in the main image.

6. An image processing device for performing a process to compose a main image of an object space to be monitored that is obtained by a main imaging device and a completing image of the object space that is imaged by a completing-purpose imaging device at the same time in a line-of-sight direction different from the main image so as to complete, when a member having a known shape is imaged in the main image together with the object space, image information on a cutoff region in the main image that is cut off by at least a portion of the member with image information on the completing image, the image processing device comprising:

set point position identifying means configured for identifying, with respect to set points which are set in the object space, sets of in-screen coordinates in a screen coordinate system of the main image and sets of in-screen coordinates in a screen coordinate system of the completing image, from three-dimensional positions of the set points and three-dimensional positions of the main imaging device and the completing-purpose imaging device;

completing image transforming means configured for generating, on the basis of the sets of in-screen coordinates, a transformed image in which pieces of image information on points in the completing image are moved in a screen of the completing image such that the completing image is converted into that in a line-of-sight direction of the main image;

cutoff region identifying means configured for identifying a position of the cutoff region in the main image; and

composite image generating means configured for generating a composite image in which a cutoff region in the main image is completed with the completing image by replacing image information on the cutoff region with image information on a corresponding region that corresponds to the cutoff region in the transformed image or superimposing the image information on the corresponding region onto the image information on the cutoff region.

7. A program for an image processing device that includes a computer for performing a process to compose a main image of an object space to be monitored that is obtained by a main imaging device and a completing image of the object space that is imaged by a completing-purpose imaging device at the same time in a line-of-sight direction different from the main image so as to complete, when a member having a known shape is imaged in the main image together with the object space, image information on a cutoff region in the main image that is cut off by at least a portion of the member with image information on the completing image, the program for the image processing device causing the computer to function by serving as:

> set point position identifying means for calculating, with respect to set points which are set in the object space, sets of in-screen coordinates of the set points in the main image and sets of in-screen coordinates of the set points in the completing image, from three-dimensional position of the set points and three-dimensional positions of the main imaging device and the completing-purpose imaging device;
> completing image transforming means for generating, on the basis of the sets of in-screen coordinates, a transformed image in which pieces of image information on points in the completing image are moved in a screen of the completing image such that the completing image is converted into that in a line-of-sight direction of the main image;
> cutoff region identifying means for identifying a position of the cutoff region in the main image; and
> composite image generating means for generating a composite image in which a cutoff region in the main image is completed with the completing image by replacing image information on the cutoff region with image information on a corresponding region that corresponds to the cutoff region in the transformed image or superimposing the image information on the corresponding region onto the image information on the cutoff region.

**Patentansprüche**

1. Bildvervollständigungssystem für einen Bildausschnittbereich, umfassend:

> eine Hauptabbildungsvorrichtung, die dazu konfiguriert ist, ein Hauptbild abzurufen, in dem ein zu überwachender Objektraum abgebildet ist;
> eine der Vervollständigung dienende Abbildungsvorrichtung, die zum Erhalten eines Vervollständigungsbildes konfiguriert ist, das zum Vervollständigen des Hauptbildes durch Abbilden des Objektraums in einer anderen Sichtlinienrichtung als die der Hauptabbildungsvorrichtung verwendet wird; und
> eine Bildverarbeitungsvorrichtung, die dazu konfiguriert ist, einen Abschnitt des Hauptbildes mit dem Vervollständigungsbild auf der Basis von dreidimensionalen Positionen von gesetzten Punkten, die in dem Objektraum gesetzt sind, und den dreidimensionalen Positionen der Hauptabbildungsvorrichtung und der der Vervollständigung dienenden Abbildungsvorrichtung zu vervollständigen,
eine Distanzmessungsvorrichtung, die zum Messen von Trennabständen zwischen den gesetzten Punkten und einem vorgegebenen Referenzpunkt konfiguriert ist; und
eine dreidimensionale Positionsmessungsvorrichtung, die zum Messen der dreidimensionalen Positionen der Hauptabbildungsvorrichtung und der der Vervollständigung dienenden Abbildungsvorrichtung konfiguriert ist,
wobei die Hauptabbildungsvorrichtung und die der Vervollständigung dienende Abbildungsvorrichtung so bereitgestellt sind, dass sie den Objektraum fast simultan abbilden,
wobei die Bildverarbeitungsvorrichtung dazu konfiguriert ist, aus dem Vervollständigungsbild Bildinformationen über einen Ausschnittsbereich in dem Objektraum zu erhalten, der hinter einer Tiefenseite eines Elements mit einer bekannten Form versteckt wird, indem das Element in dem Hauptbild zusammen mit dem Objektraum abgebildet wird, auf der Basis von Informationen über die dreidimensionalen Positionen, die kontinuierlich ermittelt werden, und dazu, die Bildinformationen über das Element in dem Hauptbild mit den erhaltenen Bildinformationen zu ersetzen oder die erhaltenen Bildinformationen auf die Bildinformationen über das Element im Hauptbild aufzulagern, um ein zusammengesetztes Bild zu erzeugen, in dem der Ausschnittsbereich mit dem Vervollständigungsbild vervollständigt ist, und
wobei die Bildverarbeitungsvorrichtung dazu konfiguriert ist, einen Abschnitt des Hauptbildes mit dem Vervollständigungsbild auf der Basis der Messergebnisse von der Distanzmessungsvorrichtung und der dreidimensionalen Positionsmessungsvorrichtung zu vervollständigen.

2. Bildvervollständigungssystem für einen Bildausschnittbereich nach Anspruch 1, wobei die Bildverarbeitungsvorrichtung beinhaltet:

> ein Identifikationsmittel für gesetzte Punkte zum Identifizieren, bezüglich der gesetzten Punkte, von Sätzen von Bildschirmkoordinaten in einem Bildschirmkoordinatensystem in dem Hauptbild und Sätzen von Bildschirmkoordinaten in einem Bildschirmkoordinatensystem in dem Vervollständigungsbild auf der Basis eines Ermittlungsergebnisse der dreidimensionalen Positionen;
> ein Vervollständigungsbildumwandlungsmittel zum Erzeugen, auf der Basis der Sätze der Bildschirmkoordinaten, eines umgewandelten Bildes, in dem Teile der Bildinformationen über Punkte in dem Vervollständigungsbild auf einem Bildschirm des Vervollständigungsbildes so verschoben sind, dass das Vervollständigungsbild zu dem Bild in einer Sichtlinienrichtung der Hauptabbildungsvorrichtung konvertiert wird; ein Ausschnittsbereichsidentifikationsmittel zum Identifizieren einer Position des Ausschnittsbereichs in dem Hauptbild; und ein Erzeugungsmittel für zusammengesetzte Bilder zum Erzeugen des zusammengesetztes Bildes durch Austauschen der Bildinformationen über den Ausschnittsbereich mit Bildinformationen über einen entsprechenden Bereich, der dem Ausschnittsbereich in dem umgewandelten Bild entspricht, oder durch Auflagern der Bildinformationen über den entsprechenden Bereich auf die Bildinformationen über den Ausschnittsbereich.

3. Bildvervollständigungssystem für einen Bildausschnittbereich nach Anspruch 1, wobei mindestens drei gesetzte Punkte in dem Objektraum gesetzt werden.

4. Bildvervollständigungssystem für einen Bildausschnittbereich nach Anspruch 1, wobei die Hauptabbildungsvorrichtung und die der Vervollständigung dienende Abbildungsvorrichtung so bereitgestellt sind, dass sie unabhängig bewegt werden können, um den Objektraum abzubilden.

5. Bildvervollständigungssystem für einen Bildausschnittbereich nach Anspruch 2, wobei das Vervollständigungsbildumwandlungsmittel das umgewandelte Bild durch Verschieben der Teile der Bildinformationen über das Vervollständigungsbild solcher Art erzeugt, dass die gesetzten Punkte in dem Vervollständigungsbild mit Sätzen von Bildschirmkoordinaten derselben gesetzten Punkte übereinstimmen, die in dem Hauptbild vorhanden sind.

6. Bildverarbeitungsvorrichtung zum Durchführen eines Prozesses zum Zusammensetzen eines Hauptbildes eines zu überwachenden Objektraums, das von einer Hauptabbildungsvorrichtung erhalten wird, und eines Vervollständigungsbildes des Objektraums, das durch eine der Vervollständigung dienenden Abbildungsvorrichtung zur selben Zeit in einer anderen Sichtlinienrichtung als die des Hauptbildes abgebildet wird, um, wenn ein Element mit einer bekannten Form in dem Hauptbild zusammen mit dem Objektraum abgebildet wird, Bildinformationen über einen Ausschnittsbereich in dem Hauptbild, der zumindest um einen Abschnitt des Elements beschnitten ist, mit Bildinformationen über das Vervollständigungsbild zu vervollständigen, wobei die Bildverarbeitungsvorrichtung umfasst:

ein Identifikationsmittel für gesetzte Punkte, das dazu konfiguriert ist, bezüglich der gesetzten Punkte, die in dem Objektraum gesetzt sind, Sätze von Bildschirmkoordinaten in einem Bildschirmkoordinatensystem des Hauptbildes und Sätze von Bildschirmkoordinaten in einem Bildschirmkoordinatensystem des Vervollständigungsbildes aus dreidimensionalen Positionen der gesetzten Punkte und dreidimensionalen Positionen der Hauptabbildungsvorrichtung und der der Vervollständigung dienenden Abbildungsvorrichtung zu identifizieren; ein Vervollständigungsbildumwandlungsmittel, das dazu konfiguriert ist, auf der Basis der Sätze der Bildschirmkoordinaten, ein umgewandeltes Bild zu erzeugen, in dem Teile der Bildinformationen über Punkte in dem Vervollständigungsbild auf einem Bildschirm des Vervollständigungsbildes so verschoben sind, dass das Vervollständigungsbild zu dem Bild in einer Sichtlinienrichtung des Hauptbildes konvertiert wird; ein Ausschnittsbereichsidentifikationsmittel, das dazu konfiguriert ist, eine Position des Ausschnittsbereichs in dem Hauptbild zu identifizieren; und ein Erzeugungsmittel für zusammengesetzte Bilder, das dazu konfiguriert ist, ein zusammengesetztes Bild zu erzeugen, in welchem ein Ausschnittsbereich in dem Hauptbild mit dem Vervollständigungsbild vervollständigt ist, durch Austauschen der Bildinformationen über den Ausschnittsbereich mit Bildinformationen über einen entsprechenden Bereich, der dem Ausschnittsbereich in dem umgewandelten Bild entspricht, oder durch Auflagern der Bildinformationen über den entsprechenden Bereich auf die Bildinformationen über den Ausschnittsbereich.

7. Programm für eine Bildverarbeitungsvorrichtung, die einen Computer zum Durchführen eines Prozesses zum Zusammensetzen eines Hauptbildes eines zu überwachenden Objektraums beinhaltet, das von einer Hauptabbildungsvorrichtung erhalten wird, und eines Vervollständigungsbildes des Objektraums, das durch eine der Vervollständigung dienenden Abbildungsvorrichtung zur selben Zeit in einer anderen Sichtlinienrichtung als die des Hauptbildes abgebildet wird, um, wenn ein Element mit einer bekannten Form in dem Hauptbild zusammen mit dem Objektraum abgebildet wird, Bildinformationen über einen Ausschnittsbereich in dem Hauptbild, der zumindest um einen Abschnitt des Elements beschnitten ist, mit Bildinformationen über das Vervollständigungsbild zu vervollständigen, wobei das Programm für die Bildverarbeitungsvorrichtung den Computer zum Betrieb durch Dienen als Folgendes veranlasst:

ein Identifikationsmittel für gesetzte Punkte zum Berechnen, bezüglich der gesetzten Punkte, die in dem Objektraum gesetzt sind, von Sätzen von Bildschirmkoordinaten der gesetzten Punkte in dem Hauptbild und Sätzen von Bildschirmkoordinaten der gesetzten Punkte in dem Vervollständigungsbild aus dreidimensionalen Positionen der gesetzten Punkte und dreidimensionalen Positionen der Hauptabbildungsvorrichtung und der der Vervollständigung dienenden Abbildungsvorrichtung;

ein Vervollständigungsbildumwandlungsmittel zum Erzeugen, auf der Basis der Sätze der Bildschirmkoordinaten, eines umgewandelten Bildes, in dem Teile der Bildinformationen über Punkte in dem Vervollständigungsbild auf einem Bildschirm des Vervollständigungsbildes so verschoben sind, dass das Vervollständigungsbild zu dem Bild in einer Sichtlinienrichtung des Hauptbildes konvertiert wird;

ein Ausschnittsbereichsidentifikationsmittel zum Identifizieren einer Position des Ausschnittsbereichs in dem Hauptbild; und

ein Erzeugungsmittel für zusammengesetzte Bilder zum Erzeugen eines zusammengesetzten Bildes, in welchem ein Ausschnittsbereich in dem Hauptbild mit dem Vervollständigungsbild vervollständigt ist, durch Austauschen der Bildinformationen über den Ausschnittsbereich mit Bildinformationen über einen entsprechenden Bereich, der dem Ausschnittsbereich in dem umgewandelten Bild entspricht, oder durch Auflagern der Bildinformationen über den entsprechenden Bereich auf die Bildinformationen über den Ausschnittsbereich.

## Revendications

1. Système d'exécution d'image pour un pan coupé dans l'image, comprenant :

un dispositif d'imagerie principale configuré pour l'obtention d'une image principale dans laquelle un espace sujet à surveiller est imagé ;
un dispositif d'imagerie d'exécution configuré pour obtenir une image d'exécution utilisée pour réaliser l'image principale par imagerie de l'espace sujet dans un sens de ligne de vision différent de celui du dispositif d'imagerie principale ; et
un dispositif de traitement d'image configuré pour réaliser une partie de l'image principale avec l'image d'exécution sur la base des positions tridimensionnelles des points définis qui sont définis dans l'espace sujet et des positions tridimensionnelles du dispositif d'imagerie principale et du dispositif d'imagerie d'exécution,
un dispositif de mesure de distance configuré pour mesurer les distances de séparation entre les points définis et un point de référence prédéterminé ; et
un dispositif de mesure de position tridimensionnelle configuré pour mesurer des positions tridimensionnelles du dispositif d'imagerie principale et du dispositif d'imagerie d'exécution,
où le dispositif d'imagerie principale et le dispositif d'imagerie d'exécution sont prévus afin d'imager pratiquement simultanément l'espace sujet,
où le dispositif de traitement d'image est configuré pour l'obtention à partir de l'image d'exécution, d'une information d'image sur un pan coupé dans l'espace sujet qui est caché derrière sur un côté de profondeur d'un élément présentant une forme connue par l'imagerie de l'élément dans l'image principale conjointement à l'espace sujet, sur la base de l'information sur les positions tridimensionnelles qui sont continuellement détectées, et pour le remplacement de l'information d'image sur l'élément dans l'image principale par l'information d'image obtenue ou surimpose l'information d'image obtenue sur l'information d'image sur l'élément dans l'image principale afin de générer une image composite dans laquelle le pan coupé est exécuté avec l'image d'exécution, et
où le dispositif de traitement d'image est configuré pour exécuter une partie de l'image principale avec l'image d'exécution sur la base des résultats de mesure provenant du dispositif de mesure de distance et du dispositif de mesure de position tridimensionnelle.

2. Système d'exécution d'image pour un pan coupé dans l'image selon la revendication 1, dans lequel le dispositif de traitement d'image inclut :

un moyen d'identification d'une position de points définis pour l'identification, par rapport aux points définis, des ensembles de coordonnées de trame incorporées dans un système de coordonnées de trame dans l'image principale et des ensembles de coordonnées de trame incorporées dans un système de coordonnées de trame dans l'image d'exécution sur la base d'un résultat de détection des positions tridimensionnelles ;
un moyen de transformation d'image d'exécution pour générer, sur la base des ensembles de coordonnées de trame incorporées, une image transformée dans laquelle les éléments d'information d'image sur les points dans l'image d'exécution sont déplacés dans une trame de l'image d'exécution de sorte que l'image d'exécution soit convertie en celle d'un sens de ligne de vision du dispositif d'imagerie principale ;
un moyen d'identification de pan coupé pour identifier une position du pan coupé dans l'image principale ; et
un moyen de génération d'image composite pour générer l'image composite en remplaçant l'information d'image sur le pan coupé par l'information d'image d'une région correspondante qui correspond au pan coupé dans l'image transformée ou en surimposant l'information d'image sur la région correspondante sur l'information d'image sur le pan coupé.

3. Système d'exécution d'image pour un pan coupé dans l'image selon la revendication 1, dans lequel au moins trois points définis sont définis dans l'espace sujet.

4. Système d'exécution d'image pour un pan coupé dans l'image selon la revendication 1, dans lequel le dispositif d'imagerie principale et le dispositif d'imagerie d'exécution sont prévus afin de se déplacer indépendamment vers l'image de l'espace sujet.

5. Système d'exécution d'image pour un pan coupé dans l'image selon la revendication 2, dans lequel le moyen de transformation d'image d'exécution génère l'image transformée en déplaçant les éléments d'information d'image sur l'image d'exécution de sorte que les points définis dans l'image d'exécution correspondent aux ensembles de coordonnées de trame incorporées des mêmes points définis existant dans l'image principale.

6. Dispositif de traitement d'image pour l'exécution d'un procédé de composition d'une image principale d'un espace sujet à surveiller qui est obtenue par un dispositif d'imagerie principale et une image d'exécution de l'espace sujet qui est imagée par un dispositif d'imagerie d'exécution au même moment dans un sens de ligne de vision différent de l'image principale afin d'exécuter, lorsqu'un élément ayant une forme connue est imagé dans l'image principale conjointement à l'espace sujet, l'information d'image sur un pan coupé dans l'image principale qui est coupé d'au moins une partie de l'élément avec l'information d'image sur l'image d'exécution, le dispositif de traitement d'image comprenant :

un moyen d'identification d'une position de points définis configuré pour l'identification, par rapport aux points définis qui sont définis dans l'espace sujet, d'ensembles de coordonnées de trame incorporées dans un système de coordonnées de trame de l'image principale et des ensembles de coordonnées de trame incorporées dans un système de coordonnées de trame de l'image d'exécution, à partir de positions tridimensionnelles des points définis et des positions tridimensionnelles du dispositif d'imagerie principale et du dispositif d'imagerie d'exécution ;
un moyen de transformation d'image d'exécution configuré pour générer, sur la base des ensembles de coordonnées de trame incorporées, une image transformée dans laquelle des éléments d'information d'image sur les points dans l'image d'exécution sont déplacés dans une trame de l'image d'exécution de sorte que l'image d'exécution soit convertie en celle dans un sens de ligne de vision de l'image principale ;
un moyen d'identification de pan coupé configuré pour l'identification d'une position du pan coupé dans l'image principale ; et
un moyen de génération d'image composite configuré pour générer une image composite dans laquelle un pan coupé dans l'image principale est exécuté par l'image d'exécution en remplaçant l'information d'image sur le pan coupé par l'information d'image sur une région correspondante qui correspond au pan coupé dans l'image transformée ou en surimposant l'information d'image sur la région correspondante sur l'information d'image sur le pan coupé.

7. Programme d'un dispositif de traitement d'image qui inclut un ordinateur pour exécuter un procédé de composition d'une image principale d'un espace sujet à surveiller qui est obtenue par un dispositif d'imagerie principale et une image d'exécution de l'espace sujet qui est imagée par un dispositif d'imagerie d'exécution au même moment dans un sens de ligne de vision différent de l'image principale afin d'exécuter, lorsqu'un élément ayant une forme connue est imagé dans l'image principale conjointement à l'espace sujet, l'information d'image sur un pan coupé dans l'image principale qui est coupé d'au moins une partie de l'élément par l'information d'image sur l'image d'exécution, le programme pour le dispositif de traitement d'image conduisant l'ordinateur à fonctionner en servant comme :

moyen d'identification de la position de points définis pour le calcul, par rapport aux points définis qui sont définis dans l'espace sujet, des ensembles de coordonnées de trame incorporées des points définis dans l'image principale et des ensembles de coordonnées de trame incorporées des points définis dans l'image d'exécution, à partir de la position tridimensionnelle des points définis et des positions tridimensionnelles du dispositif d'imagerie principale et du dispositif d'imagerie d'exécution ;

un moyen de transformation d'image d'exécution pour générer, sur la base des ensembles de coordonnées de trame incorporées, une image transformée dans laquelle les éléments d'information d'image sur les points dans l'image d'exécution sont déplacés dans une trame de l'image d'exécution de sorte que l'image d'exécution soit convertie en celle dans un sens de ligne de vision de l'image principale ;

un moyen d'identification de pan coupé pour l'identification d'une position du pan coupé dans l'image principale ; et

un moyen de génération d'image composite pour générer une image composite dans laquelle un pan coupé dans l'image principale est exécuté avec l'image d'exécution en remplaçant l'information d'image sur le pan coupé par l'information d'image sur une région correspondante qui correspond au pan coupé dans l'image transformée ou en surimposant l'information d'image sur la région correspondante sur l'information d'image sur le pan coupé.

# FIG.1

10

12  20

| DISTANCE MEASURING MEANS |

23  13

23A

22  16(11)

22

17(11)

22

S

S

S2

(Outside the body)

S2  (Inside the body)

S1  S1

19  K

14

| SET POINT POSITION IDENTIFYING MEANS | 25 |
| COMPLETING IMAGE TRANSFORMING MEANS | 26 |
| CUTOFF REGION IDENTIFING MEANS | 27 |
| COMPOSITE IMAGE GENERATING MEANS | 28 |

# FIG.2

(A)

(B)

(E)

(C)

(F)

(D)

# FIG.3

SCREEN COORDINATE SYSTEM

REFERENCE COORDINATE SYSTEM

# FIG.4

(A)

(B)

**EP 2 829 218 B1**